# EUROPÄISCHE PATENTSCHRIFT

(11) **EP 0 680 746 B1**
(45) Veröffentlichungstag und Bekanntmachung des Hinweises auf die Patenterteilung: **24.10.2001**
(21) Anmeldenummer: 95810297.2
(22) Anmeldetag: 04.05.1995
(51) Int. Cl.: A61K 7/42

(54) **Lichtschutzmittel**
Sunscreen composition
Composition de protection contre la lumière

(30) Priorität: 04.05.1994 CH 139294
(43) Veröffentlichungstag der Anmeldung: 08.11.1995
(73) Patentinhaber: Spirig Pharma AG, 4622 Egerkingen (CH)
(72) Erfinder: Juch, Rolf Dieter, CH-4612 Wangen b. Olten (CH); Birrenbach, Gerd, CH-4616 Kappel (CH)
(74) Vertreter: Braun, André, jr.

(56) Entgegenhaltungen:
- EP-A- 0 433 086
- EP-A- 0 456 460
- EP-A- 0 535 971
- EP-A- 0 559 320
- WO-A-92/13517
- WO-A-93/11742
- FR-A- 2 646 346
- GB-A- 2 206 282
- US-A- 4 695 452

## Beschreibung

Die vorliegende Erfindung betrifft ein Lichtschutzmittel. Darunter soll ein Mittel verstanden werden, das Schutz gegen Sonnenstrahlen oder andere UVA und/oder UVB emittierende Lichtquellen bietet.

Bisher werden meist chemisch wirkende UV-Strahlen absorbierende Verbindungen in Sonnenschutzmitteln verwendet. Insbesondere werden Paraaminobenzoate (PABA-Derivate), Salicylate, Zimtsäureester und Benzophenone in solche Mittel eingearbeitet. Da diese Verbindungen vor allem auch allergische Wirkungen hervorrufen können, ist ihre qualitative und quantitative Zusammensetzung von der FDA und in EC-Richtlinien vorgeschrieben. Will man einen höheren Lichtschutzfaktor (=LSF) erreichen, d.h. eine höhere Sonnenschutzwirkung des Präparats, ist dies häufig nicht möglich, ohne die vorgegebenen Grenzwerte zu überschreiten.

EP-A-0 535 971 beschreibt eine öligen Dispersion, enthaltend Zinkoxid mit einer Korngrösse von 0.005 - 0.15 Mikron und einen Dispergator, wobei der Feststoffgehalt mindestens 30 Gewichtsprozent beträgt. FR 2 646 346 beschreibt Lichtschutzmittel, welche Licht reflektierende Feststoffe in Silikonölen enthalten.

Ein Ausweg aus diesem Dilemma besteht darin, dass man physikalisch wirkende UV-Strahlenblocker anstelle der erwähnten organisch-chemischen Verbindungen verwendet. Geeignete Beispiele dafür sind ZnO und TiO₂, die jeweils wie dies auch für die chemisch wirkenden UV-Strahlen absorbierenden organisch-chemischen Verbindungen gilt - als O/W- oder - insbesondere für wasserfeste Emulsionen W/O-Emulsionen formuliert werden.

Fett und Wasser bilden für Mikroorganismen ideale Wachstumsbedingungen. Um die Emulsionen sicher zu konservieren, müssen demzufolge Konservierungsmittel und Stabilisatoren zugesetzt werden, was sich für hautempfindliche und allergisch reagierende Anwender ungünstig auswirken kann. Zudem können die verwendeten Emulgatoren zusammen mit der Sonneneinstrahlung allergische Reaktionen, wie z.B. die sogenannte Mallorca-Akne, hervorrufen. Für den Einsatz von Pigmenten in Lichtschutzmitteln sprechen dagegen deren Ungiftigkeit, biologische Akzeptanz, fehlendes Penetrationsvermögen und hohe kosmetische Qualität.

Gemäss dem Stand der Technik ist es allerdings praktisch unmöglich, insbesondere TiO₂ in Konzentrationen von mehr als 5 % der Zusammensetzung kosmetisch akzeptabel zu verarbeiten, was die Höhe des Lichtschutzfaktors beschränkt; denn je geringer die Konzentration an Pigment ist, desto niedriger ist auch der LSF. Deshalb werden Pigmente überwiegend mit chemisch wirkenden UV-Absorbern kombiniert, um einen deutlichen LSF zu erhalten.

Es wurde nun überraschend gefunden, dass man ein wasserfreies Lichtschutzmittel unter Verwendung eines ultrafeinen Pigments, wie TiO₂ oder ZnO, und einem Lipogel, enthaltend eine Kombination von mindestens zwei ausgewählten Lipiden, als Vehikel für ein kosmetisch akzeptables und stabiles Präparat erhalten kann, dessen LSF von der Art und Menge des verwendeten Pigments, aber auch von der Art und Menge der verwendeten Kombination von mindestens zwei ausgewählten Lipiden abhängig ist.

Eine Zusammensetzung der oben erwähnten Art kann bis zu 30 %, vorzugsweise aber nicht mehr als 10 %, an Pigmenten enthalten und benötigt dazu weder chemische Filtersubstanzen noch Emulgatoren, Konservierungsmittel oder Parfum. Selbstverständlich kann die Konzentration an Pigmenten auch beträchtlich tiefer sein, sofern man keinen eigentlichen Sonnenblocker, sondern eher ein Bräunungsprodukt, das die Sonnenstrahlen nicht vollkommen von der Haut fernhalten soll, herstellen will.

Trotz der - im Verhältnis zum erreichten LSF - geringen Menge an verwendeten Pigmenten kann durch Zusatz einer Kombination von mindestens zwei ausgewählten Lipiden ein überraschend hoher LSF in vitro und in vivo erzielt werden, ohne dass zusätzlich ein chemisch wirkender UV-Absorber zugesetzt wird.

Ferner wird durch das neue Mittel der sogenannte Paniereffekt, d.h. das Anhaften z.B. von Sand am eingeriebenen Körper, vermieden. Zudem ist die erfindungsgemässe Zusammensetzung wasserbeständig.

Vorzugsweise wird ultrafeines Pigment in einem Korngrössenbereich von <100 nm verwendet, wodurch trotz hoher Konzentration die Transparenz auf der Haut gewährleistet wird. Die Agglomerationstendenz dieser kleinen Partikel wird durch die erfindungsgemässe Zusammensetzung verhindert.

Die Pigmente sind auf dem Markt erhältlich. So kann man als ultrafeines TiO₂ z.B. Tioveil von Tioxide Chemicals Limited, UK, oder Tri-K von Tri-K Industries, Inc., Emerson N.J., P25 von Degussa, MT150W von Teikoku Kako Co.Ltd., TiO₂ von Bayer oder UV-Titan von Kemira, Finnland verwenden.

Als ZnO kann man z.B. Z-Cote von Sunsmart Inc., USA verwenden.

Als Pigment kann man z.B. TiO₂ oder ZnO allein oder in Kombination miteinander benützen, wobei TiO₂ bevorzugt wird.

Durch die Konzentration von bis zu 30% an Pigmenten kann nicht nur ein absoluter Lichtschutzfaktor, sondern auch ein ausgeglichener UVA- und UVB-Schutz erreicht werden, was für die Qualität eines Lichtschutzmittels von ebenso grosser Bedeutung ist.

Der auf den Sonnenschutzpräparaten angegebene Faktor bezieht sich auf den Schutz gegen UVB-Strahlung, welche für den Sonnenbrand verantwortlich ist. Der LSF gibt an, wievielmal länger man sich der Strahlung aussetzen kann, bis erste Anzeichen eines Sonnenbrandes entstehen. Ein Sonnenschutzmittel mit LSF 20 bedeutet somit, dass sich ein Individuum 20 mal länger als in ungeschütztem Zustand z.B. der Sonne aussetzen kann.

Chronisch hohe UVA-Dosen sind hauptsächlich für die Hautalterung und die Krebsentstehung mitverantwortlich. Benützt man ein Sonnenschutzmittel nur mit hohem LSF für UVB-Strahlung, kann es vorkommen, dass die Haut zuviel UVA-Strahlung abbekommt, ohne dass dies sofort bemerkt wird. Deshalb sind Sonnenschutzmittel mit unausgewogenem Verhältnis zwischen UVA/UVB-Schutz zur Verhinderung von Langzeitschäden ungeeignet.

Das Verhältnis der gesamten Lichtreflexion/Lichtabsorption des UVA-Bereichs zum UVB-Bereich wird als Quotient unter der Fläche der UVA-Schutz-Kurve eines Lichtschutzmittels durch die Fläche der UVB-Schutzkurve bestimmt. Dabei wird ein Verhältnis von 0,2-0,4 als mässig, 0,4-0,6 als gut, 0,6-0,8 als sehr gut und >0,8 als maximal bewertet.

Das Verhältnis des erfindungsgemässen Lichtschutzmittels bewegt sich im Bereich von 0,6-0,8 und ist deshalb als sehr gut einzustufen.

Zur Herstellung eines Lipogels wird ein Gerüst benötigt, das eine Kombination von mindestens zwei ausgewählten Lipiden, nämlich von einem ersten Lipid und einem zweiten Co-Lipid, mindestens einem Konsistenzgeber, mindestens einem kosmetisch akzeptablen flüchtigen Träger und gegebenenfalls mindestens einem Suspendierhilfsmittel enthält.

Die Aufgabe des kosmetisch akzeptablen flüchtigen Trägers besteht darin, die Viskosität des Sonnenschutzmittels zu verringern, das Verstreichen und Verteilen des Sonnenschutzmittels auf der Haut zu verbessern und damit die benötigte Schichtdicke von Pigment und Kombination aus mindestens zwei Lipiden zu verringern. Hingegen besitzt der Träger keinen eigenen LSF und verflüchtigt sich nach Applikation rasch auf der Haut.

Durch die Kombination erstes Lipid, zweites Co-Lipid und flüchtiger Träger wird der Lichtschutzfaktor erhöht, die Stabilität des Lichtschutzmittels verbessert, der Panierffekt vermieden und die notwendige kosmetische Akzeptanz erreicht.

Als erstes Lipid eignen sich Fettsäureester, wie Ethylhexylpalmitat Octylstearat, Diisopropylsebacat, C₁₂-C₁₅ Alkoholbenzoate, Ethylhexylstearat, Diisopropyldimerat, Myristyllaktat oder Isopropylmyristat, oder Gemische davon.

Als zweites Co-Lipid eignen sich Polysiloxan-Polyalkylen-Copolymere, pflanzliche Öle und daraus gewonnene Ölfraktionen, wie Jojobaöl, Sonnenblumenöl, Mandelöl, Distelöl, Passionsblumenöl, Sheabutter, Sojaöl, Weizenkeimöl, Olivenöl, C ₈-C₁₀ Triglyceride oder Gemische davon.

Als kosmetisch akzeptable flüchtige Träger eignen sich Wundbenzin, volatile Silikone, wie z.B. Cyclometicon, Dimeticon, Phenyldimeticon, Cyclohexan, Ethylpropionat, Methylethylketon, Ethylglykolmethylether, Butylacetat, Trichlorethan, Shellsol T, Ethyllactat oder Gemische davon.

Als Konsistenzgeber eignen sich wachsartige Substanzen, wie z.B. Ozokerit, Carnabauwachs, Bienenwachs, Hydroxyoctacosanyl Hydroxystearat, Paraffinwachs, Glyceryltribehenat, C₁₈-C₃₆ Glykolestersäuren, partiell hydrierte C₁₄-C₂₀ Glyceride, Zinkstearat, Aluminiumstearat oder Calciumstearat oder Gemische davon.

Als das Absinken von Teilchen verhinderndes Suspendierhilfsmittel eignen sich z.B. Lecithin oder kolloidales Siliciumdioxid oder ein Gemisch davon.

Bei Bedarf kann man ein Antioxidans, wie z.B. dl-alpha-Tocopherol, Butylhydroxyanisol oder Ascorbylpalmitat zugeben.

Das erfindungsgemässe Lichtschutzmittel enthält bezogen auf sein Gesamtgewicht vorzugsweise:
1-30%, vorzugsweise 5-15%, besonders bevorzugt 10% eines anorganischen Pigments, wie ultrafeines TiO₂ oder ZnO, oder eines Gemisches davon,
5-30%, vorzugsweise 10-25%, besonders bevorzugt 19% eines ersten Lipids oder eines Gemisches davon,
1-20%, vorzugsweise 5-15%, besonders bevorzugt 10% eines zweiten Co-Lipids oder eines Gemisches davon,
1-80%, vorzugsweise 30-60%, besonders bevorzugt 53% eines kosmetisch akzeptablen flüchtigen Trägers oder eines Gemisches davon,
1-20%, vorzugsweise 3-15%, besonders bevorzugt 6% eines Konsistenzgebers oder eines Gemisches davon,
0-10%, vorzugsweise 1-7%, besonders bevorzugt 2% eines Suspendierhilfsmittels oder eines Gemisches davon,
0-5%, vorzugsweise 0,05-3%, besonders bevorzugt 0,1% eines Antioxidans oder eines Gemisches davon.

Das Lichtschutzmittel kann je nach Zusammensetzung von lotioartiger oder crèmeartiger Konsistenz sein, ist aber wegen der Abwesenheit von Wasser eine O/W- oder W/O-Emulsion.

Die Erfindung wird im folgenden an Hand von Beispielen näher erläutert.

### Beispiel 1

Es wird ein Lichtschutzmittel crèmeartiger Konsistenz folgender Zusammensetzung hergestellt:

| | |
|---|---|
| Titandioxid ultrafein | 10,0 g |
| Ethylhexylpalmitat | 19,0 g |
| Passionsblumenöl | 10,0 g |
| Cyclometicon | 41,0 g |
| Dimeticon | 10,0 g |
| Carnaubawachs | 10,0 g |

Alle Stoffe mit Ausnahme des Titandioxids und des flüchtigen Trägers werden zusammengegeben und auf 80°C erhitzt. Darauf wird der Pigmentanteil unter Rühren eingegeben und auf 40°C abgekühlt. Danach wird der flüchtige Träger unter Kühlen und Mischen zugefügt und das Gemisch auf 25°C abgekühlt.

### Beispiel 2

Es wird ein Lichtschutzmittel crèmeartiger Konsistenz folgender Zusammensetzung hergestellt:

| | |
|---|---|
| Titandioxid ultrafein | 10,0 g |
| Ethylhexylpalmitat | 10,0 g |
| Polysiloxan-Polyalkylen-Copolymer | 10,0 g |
| Cyclometicon | 49,5 g |
| Dimeticon | 10,0 g |
| Hydroxyoctacosanyl Hydroxystearat | 10,0 g |
| Lecithin | 0,5 g |

Das Herstellungsverfahren wird analog zu dem im Beispiel 1 beschriebenen durchgeführt.

### Beispiel 3

Es wird ein Lichtschutzmittel crèmeartiger Konsistenz folgender Zusammensetzung hergestellt:

| | |
|---|---|
| Titandioxid ultrafein | 10,0 g |
| C₁₂-C₁₅ Alkoholbenzoat | 15,0 g |
| Jojobaöl | 10,0 g |
| Cyclometicon | 45,0 g |
| Dimeticon | 10,0 g |
| Ozokerit | 5,0 g |
| partiell hydrierte C₁₄-C₂₀ Glyceride | 5,0 g |

Das Herstellungsverfahren wird analog zu dem im Beispiel 1 beschriebenen durchgeführt.

### Beispiel 4

Es wird ein Lichtschutzmittel lotioartiger Konsistenz folgender Zusammensetzung hergestellt:

| | |
|---|---|
| Titandioxid ultrafein | 10,0 g |
| Ethylhexylpalmitat | 19,0 g |
| Mandelöl | 10,0 g |
| Cyclometicon | 42,9 g |
| Dimeticon | 10,0 g |
| Carnaubawachs | 3,0 g |
| partiell hydrierte C₁₄-C₂₀ Glyceride | 3,0 g |
| kolloidales Siliciumdioxid | 2,0 g |
| dl-alpha-Tocopherol | 0,1 g |

Das Herstellungsverfahren wird analog zu dem im Beispiel 1 beschriebenen durchgeführt.

### Beispiel 5

Es wird ein Lichtschutzmittel lotioartiger Konsistenz folgender Zusammensetzung hergestellt:

| | |
|---|---|
| Titandioxid ultrafein | 8,0 g |
| Zinkoxid ultrafein | 2,0 g |
| Diisopropylsebacat | 19,0 g |
| Distelöl | 10,0 g |
| Dimeticon | 10,0 g |
| Carnaubawachs | 3,0 g |
| partiell hydrierte C₁₄-C₂₀ Glyceride | 3,0 g |
| kolloidales Siliciumdioxid | 2,0 g |
| dl-alpha-Tocopherol | 0,1 g |

Das Herstellungsverfahren wird analog zu dem im Beispiel 1 beschriebenen durchgeführt.

### Beispiel 6

Es wird ein Lichtschutzmittel lotioartiger Konsistenz folgender Zusammensetzung hergestellt:

| | |
|---|---|
| Titandioxid ultrafein | 10,0 g |
| Zinkoxid ultrafein | 5,0 g |
| Ethylhexylstearat | 25,0 g |
| Weizenkeimöl | 10,0 g |
| Cyclometicon | 39,5 g |
| Hydroxyoctacosanyl Hydroxystearat | 5,0 g |
| partiell hydrierte C₁₄-C₂₀ Glyceride | 2,0 g |
| kolloidales Siliciumdioxid | 3,0 g |
| Lecithin | 0,5 g |

Das Herstellungsverfahren wird analog zu dem im Beispiel 1 beschriebenen durchgeführt.

### Beispiel 7

Es wurden die Lichtschutzfaktoren von verschiedenen Kombinationen von erstem Lipid und zweitem Co-Lipid mit Titandioxid in einem in vitro-Versuch nach der Methode von Diffey (Lit.: A new substrate to measure sunscreen protection factors throughout the ultraviolet spectrum, B.L. Diffey and J. Robson, J. Soc. Cosmet. Chem., 40, 127-133 [May/June 1989]) untersucht. Das Co-Lipid ist auf der senkrechten Achse der verschiedenen Diagramme (1-5) eingezeichnet. Für jedes Diagramm wird ein anderes erstes Lipid verwendet. Verwendet wurde jeweils eine Kombination von 10% TiO₂, 15% erstem Lipid, 10% zweitem Co-Lipid, 45% Cyclometicon, 10% Dimeticon und je 3% Carnaubawachs und C₁₄-C₂₀ Glyceriden. Daraus wird ersichtlich, dass der Lichtschutzfaktor je nach Kombination des ersten Lipid und zweiten Co-Lipids bei gleichbleibender Menge TiO₂ beträchtliche Unterschiede aufweist.

### Beispiel 8

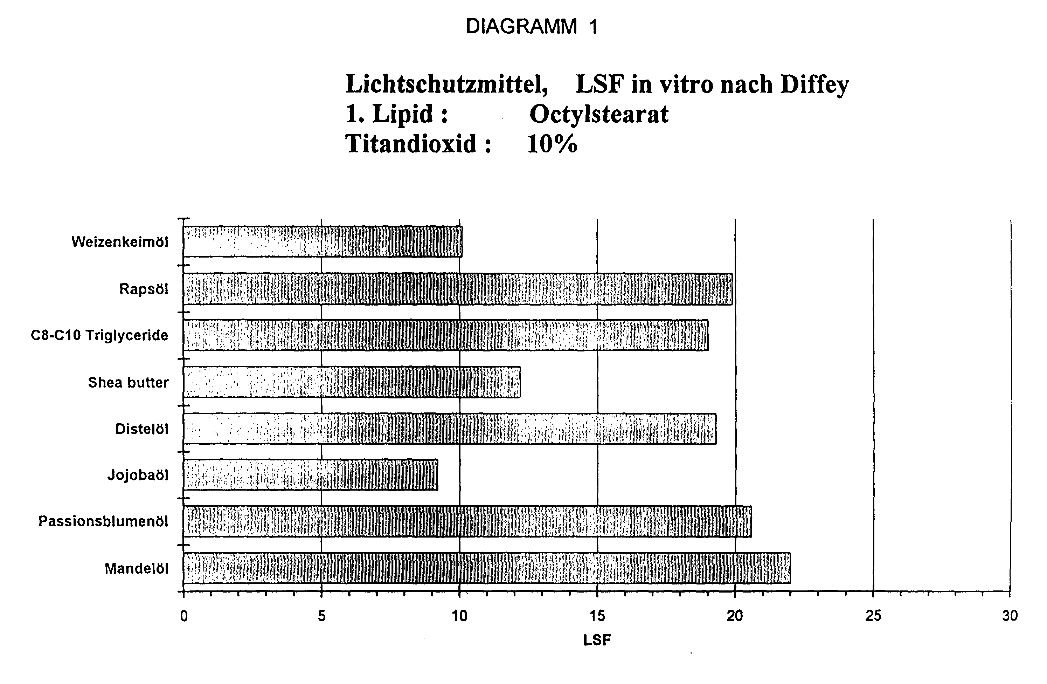

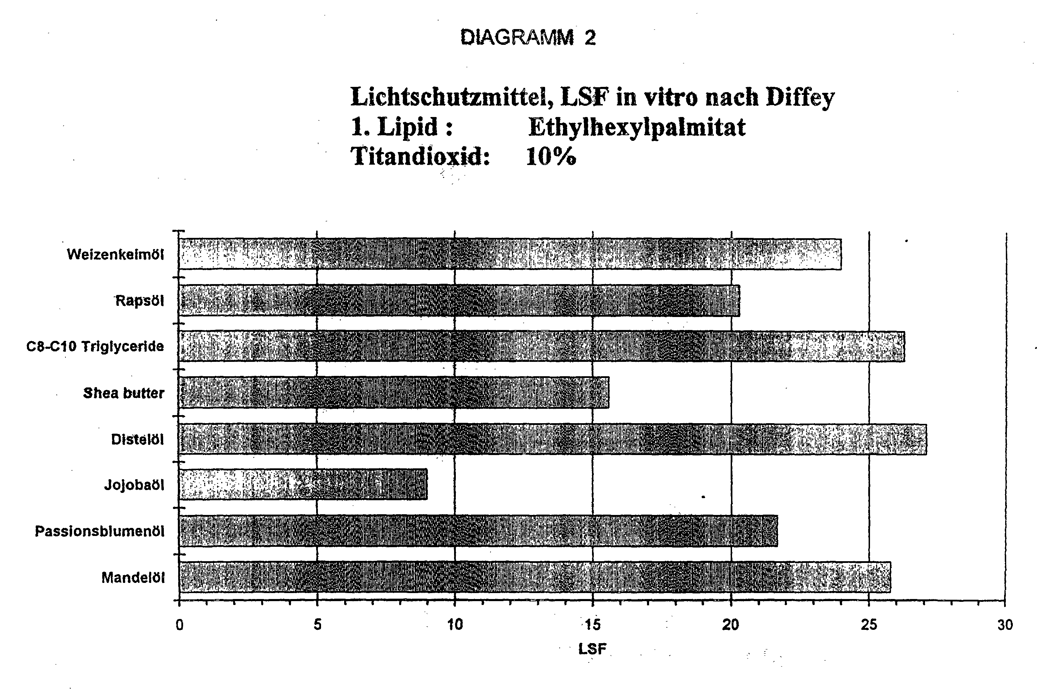

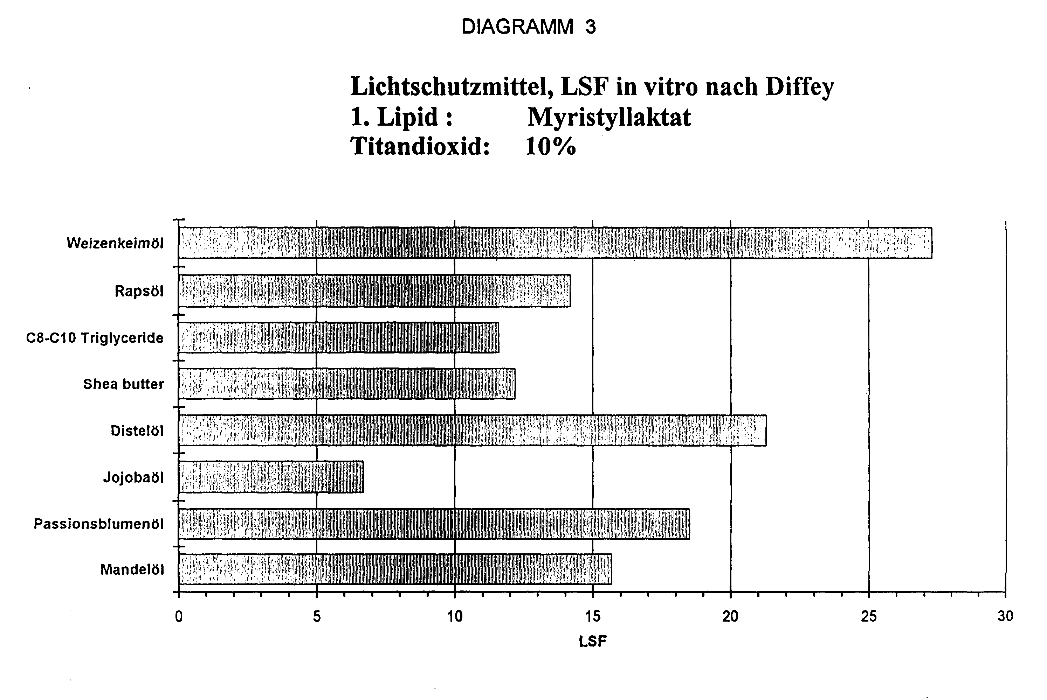

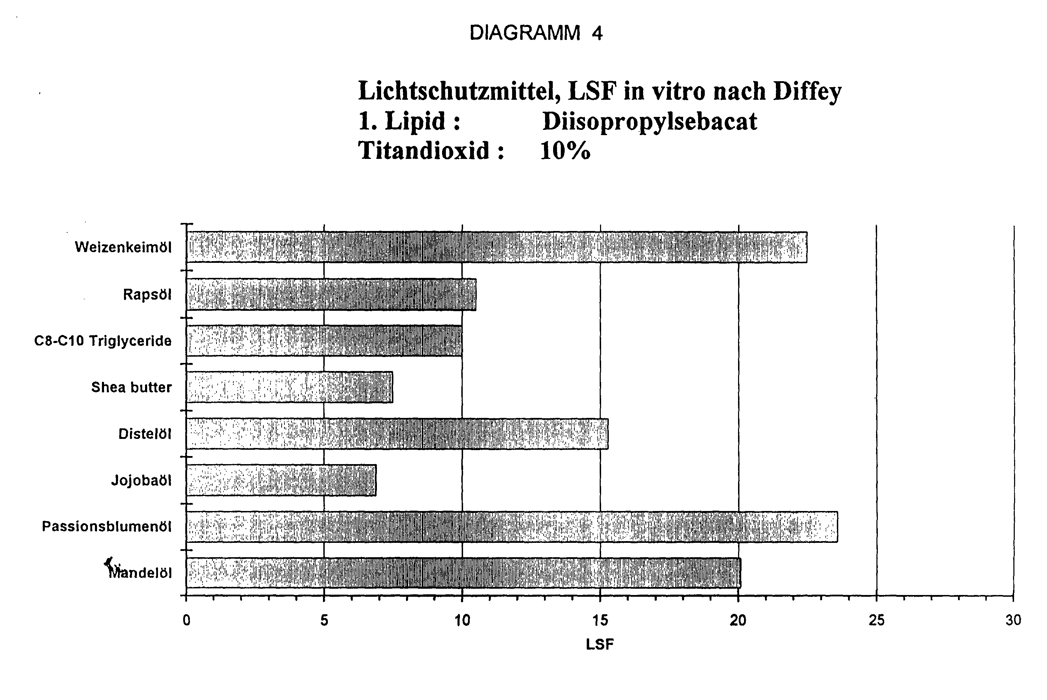

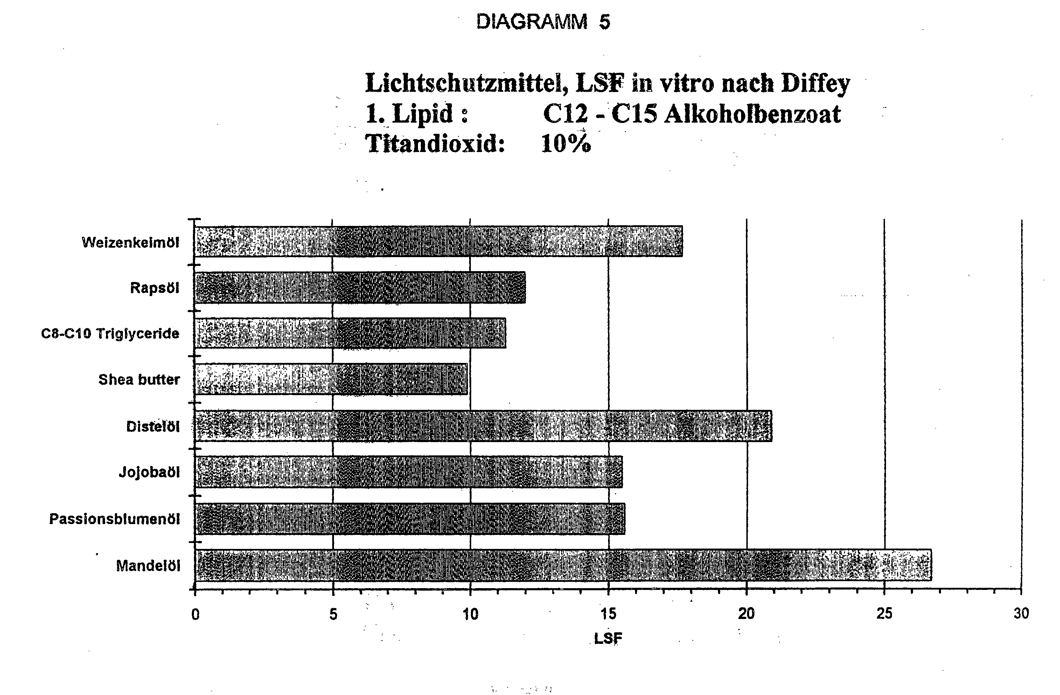

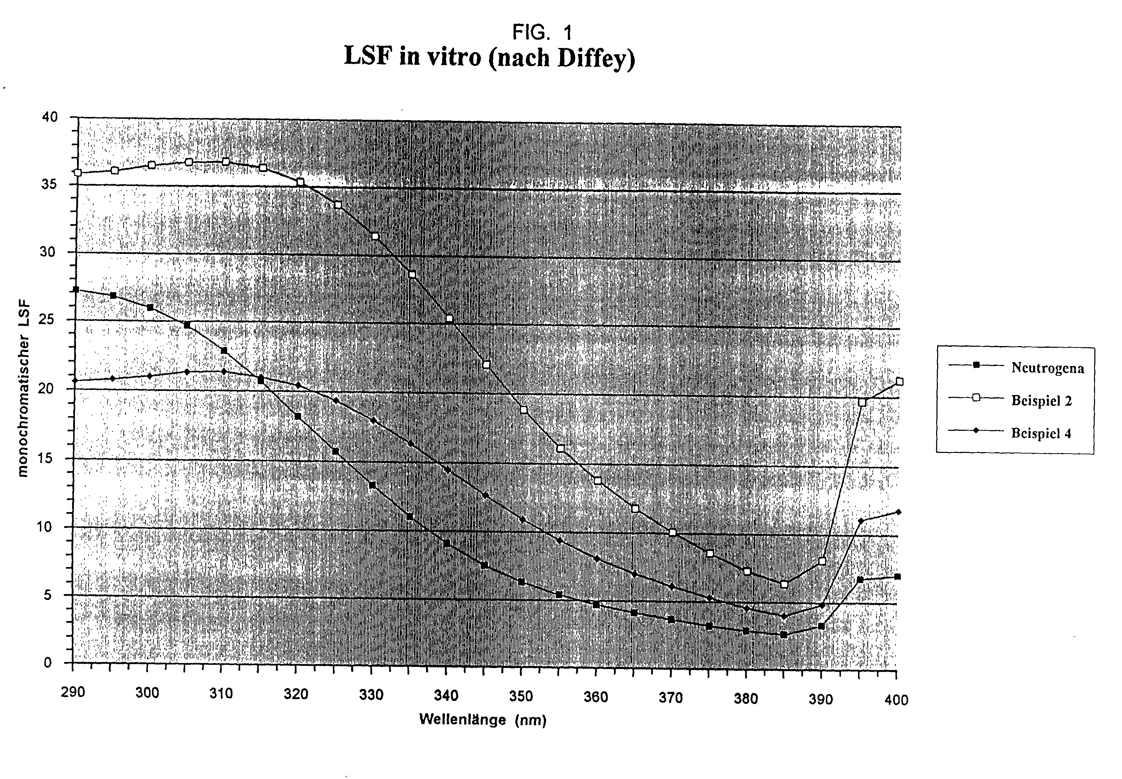

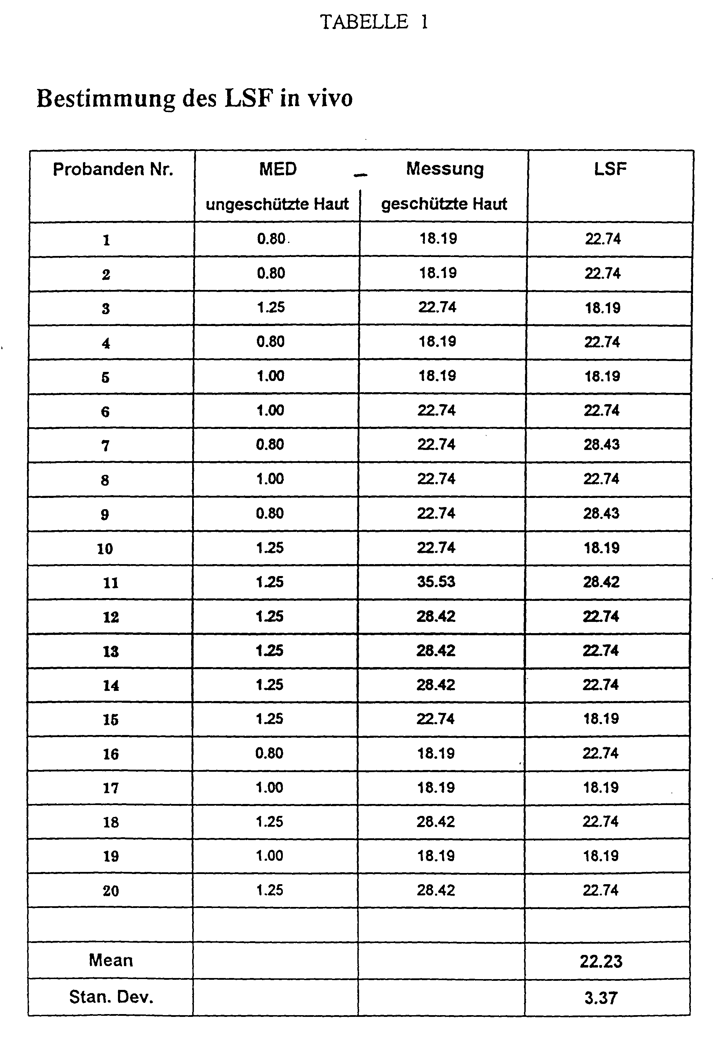

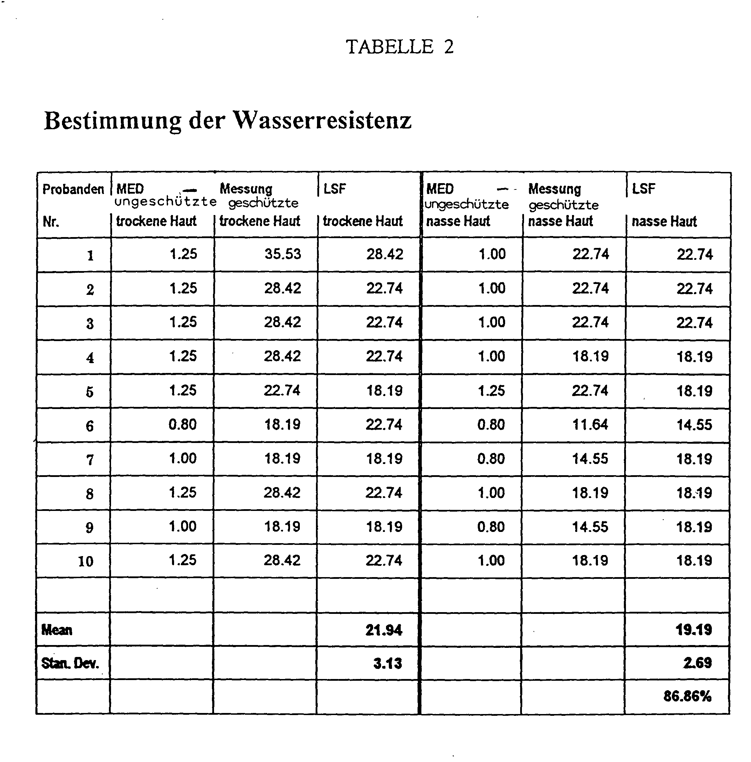

In einem in vitro Vergleichsversuch nach der Methode von Diffey wurden die Lichtschutzfaktoren des käuflichen Produkts Neutrogena^{R} mit Titandioxid als alleinigem Lichtschutzmittel mit der erfindungsgemässen Formulierung gemäss den Beispielen 2 und 4 verglichen. In Fig.1 werden die günstigen Lichtschutzfaktoren der erfindungsgemässen Formulierungen und das ausgeglichene Verhältnis des Schutzes gegenüber UVA- und UVB-Strahlen gegenüber dem Handelspräparat im in vitro-Test nach Diffey ersichtlich. Ein Ausmessen des Verhältnisses des nach Beispiel 4 hergestellten Sonnenschutzmittels ergibt ein Resultat von ca. 0,7, was als sehr gut bewertet wird.

### Beispiel 9

Am Laboratoire de Recherches sur les Tumeurs de la Peau Humaine in Paris wurde das Lichtschutzmittel nach Beispiel 4 in vivo an 20 Probanden geprüft. Die Bestimmung des LSF in vivo erfolgte nach einer FDA-Methode und Empfehlungen der CIE mit einer 150 Watt Xenon UV-Lampe und einem Robertson-Berger-Dosimeter zur Messung der Erythemschwellenzeit der Testpersonen (minimale erythemale Dosis für die geschützte resp. ungeschützte Haut = MED). In der Tabelle 1 werden die hohen gemessenen LSF der einzelnen Probanden ersichtlich.

### Beispiel 10

Die Wasserresistenz des Lichtschutzmittels nach Beispiel 4 wurde am Laboratoire de Recherches sur les Tumeurs de la Peau Humaine in Paris an 10 Probanden gemessen. Die Testfläche auf dem Rücken der Probanden betrug 100 cm². Die Messung des LSF erfolgte vor und nach dem Schwimmen (2x20 min) in einem Whirlpool in 32°C warmem Wasser. In der Tabelle 2 werden die hohen gemessenen LSF vor und nach dem Schwimmen ersichtlich. Die sehr gute Wasserresistenz des erfindungsgemässen Lichtschutzmittels ist an der sehr geringen Reduktion des LSF nach dem Schwimmen ersichtlich.

## Patentansprüche

1. Ein im wesentlichen wasserfreies Lichtschutzmittel, enthaltend mindestens ein ultrafeines Pigment und ein Lipogel, wobei dieses Lipogel eine Kombination eines Lipids und eines Co-lipids ist, **dadurch gekennzeichnet dass** diese Lichtschutzmittel die folgenden Bestandteile enthält:
a) ein ultrafeines Pigment, vorzugsweise TiO₂ und/oder ZnO in einem Korngrössenbereich von <100nm und einer Konzentration von 1-30 Gew.-% bezogen auf das Gesamtgewicht der Zusammensetzung;
b) ein Lipid ausgewählt von Fettsäureestern, in einer Konzentration von 5-30 Gew.-% bezogen auf das Gesamtgewicht der Zusammensetzung;
c) eine Co-Lipid ausgewählt aus Polysiloxanepolyalkylene-copolymeren, pflanzlichen Ölen und daraus entnommenen Ölfraktionen, in einer Konzentration von 1-20 Gew.-% bezogen auf das Gesamtgewicht der Zusammensetzung;
d) mindestens ein Konsistenzgeber ausgewählt aus wachsartigen Substanzen, in einer Konzentration von 1-20 Gew.-% bezogen auf das Gesamtgewicht der Zusammensetzung;
e) mindestens ein kosmetisch akzeptabler flüchtiger Träger, in einer Konzentration von 1-80 Gew.-% bezogen auf das Gesamtgewicht der Zusammensetzung;
f) gegebenenfalls mindestens ein Suspendierhilfsmittel, in einer Konzentration von 0-10 Gew.-% bezogen auf das Gesamtgewicht der Zusammensetzung;
g) gegebenenfalls mindestens ein Antioxidans, in einer Konzentration von 0-5 Gew.-%, bezogen auf das Gesamtgewicht der Zusammensetzung.

2. Lichtschutzmittel gemäss Anspruch 1, worin das Lipid ausgewählt ist aus Ethylhexylpalmitat, Octylstearat, Diisopropylsebacat, (C₁₂-C₁₅)-Alkoholbenzoate, Ethylhexylstearate, Diisopropyldimerat, Myristyllaktylat, Isopropylmyristat, oder Gemische davon.

3. Lichtschutzmittel nach Anspruch 1 oder 2, worin das Co-Lipid ausgewählt ist aus Jojobaöl, Sonnenblumenöl, Mandelöl, Distelöl, Passionsblumenöl, Sheabutter, Sojaöl, Weizenkeimöl, Olivenöl, (C₈-C₁₀)-Triglyceriden or Gemische davon.

4. Lichtschutzmittel nach einem der Ansprüche 1 bis 3, worin der Konsistenzgeber ausgewählt ist Ozokerit, Carnaubawachs, Bienenwachs, Hydroxyoctacosanyl Hydroxystearat, Paraffinwachs, Glyceryltribehenat, (C₁₈-C₃₆)-Glykolestersäuren, partiell hydrierte (C₁₄-C₂₀)-Glyceride, Zinkstearat, Aluminiumstearat, Calciumstearate, oder Gemische davon.

5. Lichtschutzmittel nach einem der Ansprüche 1 bis 4, worin der flüchtige Träger ausgewählt ist aus Wundbenzin, volatilen Silikonen, vorzugsweise Cyclomethicon, Dimethicon, Phenyldimethicon, Cyclohexan, Ethylpropionat, Methylethylketon, Ethylglykolmethylether, Butylacetat, Trichlorethan, Shellsol® T, Ethyllactat oder Gemische davon.

6. Lichtschutzmittel nach einem der Ansprüche 1 bis 5, worin das Suspendierhilfsmittel ausgewählt ist aus Lecithin oder kolloidalem Siliciumdioxid oder einem Gemisch derselben.

7. Lichtschutzmittel nach einem der Ansprüche 1 bis 6, worin das Antioxidans ausgewählt ist aus dl-alpha-Tocopherol, Butylhydroxyanisol oder Ascorbylpalmitate.

8. Lichtschutzmittel nach einem der Ansprüche 1 bis 8, worin jeweils
a) das ultrafeine Pigment in einer Konzentration von 5-15 Gew.-%;
b) das Lipid in einer Konzentration von 10-25 Gew.-%;
c) das Co-Lipid in einer Konzentration von 5-15 Gew.-%;
d) der Konsistenzgeber in einer Konzentration von 3-15 Gew.-%;
e) der kosmetisch akzeptable flüchtige Träger in einer Konzentration von 30-60 Gew.-%;
f) das Suspendierhilfsmittel in einer Konzentration von 1-7 Gew.-%; und
g) das Antioxidans in einer Konzentration von 0.05-3 Gew.-% anwesend ist.

9. Lichtschutzmittel nach einem der Ansprüche 1 bis 8, worin jeweils
a) das ultrafeine Pigment in einer Konzentration von 10 Gew.-%;
b) das Lipid in einer Konzentration von 19 Gew.-%;
c) das Co-Lipid in einer Konzentration von 10 Gew.-%;
d) der Konsistenzgeber in einer Konzentration von 6 Gew.-%;
e) der kosmetisch akzeptable flüchtige Träger in einer Konzentration von 53 Gew.-%;
f) das Suspendierhilfsmittel in einer Konzentration von 2 Gew.-%, und
g) das Antioxidans in einer Konzentration von 0.1 Gew.-% anwesend ist.

10. Lichtschutzmittel nach einem der Ansprüche 1 bis 9, worin dieses 10 Gew.-% Titandioxid ultrafein, 19 Gew.-% Ethylhexylpalmitat, 10 Gew.-% Mandelöl, 42,9 Gew.- %Cyclometicon, 10 Gew.-% Dimethicon, 3 Gew.-% Carnaubawachs, 3 Gew.-% partiell hydriertes C₁₄-C₂₀ Glyceride, 2 Gew.-% kolloidales Siliciumdioxid und 0.1 Gew.-% dl-alpha-Tocopherol enthält.

## Claims

1. An essentially water-free light protection preparation, comprising at least an ultra-fine pigment and a lipogel, said lipogel being a combination of a lipid and a co-lipid, **characterized in that** said preparation comprises:
a) an ultra-fine pigment, preferably TiO₂ and/or ZnO within a granular size range of <100 nm and in a concentration of 1-30% by weight calculated to the entire weight of the preparation;
b) a lipid selected from fatty acid esters, in a concentration of 5-30% by weight calculated to the entire weight of the preparation;
c) a co-lipid selected from polysiloxane-polyalkylene-copolymers, vegetable oils and oil fractions obtained therefrom, in a concentration of 1-20% by weight calculated to the entire weight of the preparation;
d) at least one consistency provider selected from wax like substances, in a concentration of 1-20% by weight calculated to the entire weight of the preparation;
e) at least one cosmetically acceptable volatile carrier, in a concentration of 1-80% by weight calculated to the entire weight of the preparation;
f) optionally at least one suspension auxiliary agent, in a concentration of 0-10% by weight calculated to the entire weight of the preparation;
g) optionally at least one antioxidant, said antioxidant, in a concentration of 0-5% by weight calculated to the entire weight of the preparation.

2. A light protection preparation according to claim 1, wherein the lipid is selected from ethylhexyl palmitate, octyl stearate, di-isopropyl sebacate, (C₁₂-C₁₅)-alcoholbenzoate, ethylhexyl stearate, diisopropyl dimerate, myristyl lactylate, isopropyl myristate, or mixtures thereof.

3. A light protection preparation according to claim 1 or claim 2, wherein the co-lipid is selected from jojoba oil, sunflower oil, almond oil, thistle oil, passionflower oil, sheabutter, soy oil, wheatgerm oil, olive oil, (C₈―C₁₀)-triglycerides or mixtures thereof.

4. A light protection preparation according to any one of the claims 1 to 3, wherein the consistency provider is selected from ozocerite, carnauba wax, beeswax, hydroxyoctacosanyl hydroxystearate, paraffin wax, glyceryl tribehenate, (C₁₈-C₃₆)-glycolester acids, partially hydrated (C₁₄-C₂₀)-glycerides, zinc stearate, aluminium stearate, calcium stearate, or mixtures thereof.

5. A light protection preparation according to any one of the claims 1 to 4, wherein the volatile carrier is selected from surgical spirits, volatile silicones, preferably from cyclomethicone, dimethicone, phenyldimethicone, cyclohexane, ethylpropionate, methylethyl ketone, ethylglycolmethylether, butyl acetate, trichlorethane, Shellsol T®, ethyl lactate or mixtures thereof.

6. A light protection preparation according to any one of the claims 1 to 5, wherein the suspension auxiliary agent is selected from lecithin or colloidal silicon dioxide or a mixture thereof.

7. A light protection preparation according to any one of the claims 1 to 6, wherein the antioxidant is selected from dl-alpha-tocopherol, butyl hydroxyanisol or ascorbyl palmitate.

8. A light protection preparation according to any one of the claims 1 to 8, wherein
a) the ultra-fine pigment is present in a concentration of 5-15% by weight;
b) the lipid is present in a concentration of 10-25% by weight;
c) the co-lipid is present in a concentration of 5-15% by weight;
d) the consistency provider is present in a concentration of 3-15% by weight;
e) the cosmetically acceptable volatile carrier is present in a concentration of 30-60% by weight;
f) the suspension auxiliary agent is present in a concentration of 1-7% by weight; and
g) the antioxidant is present in a concentration of 0.05-3% by weight.

9. A light protection preparation according to any one of the claims 1 to 8, wherein
a) the ultra-fine pigment is present in a concentration of 10% by weight;
b) the lipid is present in a concentration of 19% by weight;
c) a co-lipid is present in a concentration of 10% by weight;
d) the consistency provider is present in a concentration of 6% by weight;
e) the cosmetically acceptable volatile carrier is present in a concentration of 53% by weight;
f) the suspension auxiliary agent is present in a concentration of 2% by weight; and
g) the antioxidant is present in a concentration of 0.1% by weight.

10. A light protection agent according to any one of the claims 1 to 9, containing 10 % by weight of titanium dioxide ultra fine, 19 % by weight of ethylhexyl palmitate, 10 % by weight of almond oil, 42.9 % by weight of cyclometicone, 10 % by weight of dimethicone, 3 % by weight of carnauba wax, 3 % by weight of partially hydrated (C₁₄-C₂₀)-glycerides, 2 % by weight of colloidal silicon dioxide, and0.1 % by weight of dlalpha-tocopherol.

## Revendications

1. Un agent de stabilité à la lumière essentiellement anhydre, comprenant au moins un pigment ultrafin et un lipogel, où ledit lipogel est une combinaison d'un lipide et d'un co-lipide, **caractérisé en ce que** cet agent de stabilité à la lumière contient les composants suivants:
a) un pigment ultrafin, de préférence TiO₂ et/ ou ZnO dans une zone granulométrique de < 100 nm et en une concentration de 1-30 % en poids par rapport au poids total de la composition;
b) un lipide choisi parmi des esters d'acides gras, en une concentration de 5-30 % en poids par rapport au poids total de la composition;
c) un co-lipid choisi parmi des polysiloxane- polyalcoylène- copolymères, des huiles végétales et des fractions d'huile qu'on en obtient, en une concentration de 1-20 % en poids par rapport au poids total de la composition;
d) au moins un donneur de consistance choisi parmi des substances cireuses, en une concentration de 1-20 % en poids par rapport au poids total de la composition;
e) au moins un porteur volatil cosmétiquement acceptable, en une concentration de 1-80 % en poids par rapport au poids total de la composition;
f) optionnellement au moins un agent auxiliaire de mise en suspension en une concentration de 0-10 % en poids par rapport au poids total de la composition;
g) optionnellement au moins un antioxydant, en une concentration de 0-5 % en poids par rapport au poids total de la composition.

2. Un agent de stabilité à la lumière selon la revendication 1, où le lipide est choisi parmi l'éthyl-hexyl-palmitate, octylstéarate, diisopropyl-sébacate, (C₁₂-C₁₅)-alcool-benzoate, éthyl-hexyl-stéarate, diisopropyl-dimérate, myristyle-lactylate, isopropyl-myristate ou leurs mélanges.

3. Un agent de stabilité à la lumière selon la revendication 1 ou 2, où le co-lipide est choisi parmi l'huile de jojoba, huile de tournesol, huile d'amandes, huile de chardons, huile de fleur de la passion, beurre de shea, huile de soja, huile de germes de blé, huile d'olive, (C₈-C₁₀)-triglycérides ou leurs mélanges.

4. Un agent de stabilité à la lumière selon l'une quelconque des revendications 1 à 3, où le donneur de consistance est choisi parmi l'ozocérite, cire de carnauba, cire d'abeille, hydroxyoctacosanyle hydroxystéarate, cire de paraffine, glycéryl-tribéhenat, (C₁₈-C₃₆)-acides d'ester glycolique, (C₁₄-C₂₀)-glycérides partiellement hydrogénés, stéarate de zinc, stéarate d'aluminium, stéarate de calcium ou leurs mélanges.

5. Un agent de stabilité à la lumière selon l'une quelconque des revendications 1 à 4, où le porteur volatil est choisi parmi l'essence chirurgicale, silicons volatils, de préférence cyclométhicon, diméthicon, phényldiméthicon, cyclohexane, éthylpropionate, méthyl-éthyl-cétone, éthylglycol-méthyl-éther, butyl-acétate, trichloréthane, shellsol® T, éthyllactate ou leurs mélanges.

6. Un agent de stabilité à la lumière selon l'une quelconque des revendications 1 à 5, où l'agent auxiliaire de mise en suspension est choisi parmi la lécithine ou le dioxyde de silicium colloïdal ou leurs mélanges.

7. Un agent de stabilité à la lumière selon l'une quelconque des revendications 1 à 6, où l'antioxydants est choisi parmi le dl-alpha-tocophérol, butyl-hydroxyanisol ou ascorbyl-palmitate.

8. Un agent de stabilité à la lumière selon l'une quelconque des revendications 1 à 8, où sont présents:
a) le pigment ultrafin en une concentration de 5-15 % en poids;
b) le lipide en une concentration de 10-25 % en poids;
c) le co-lipide en une concentration de 5-15 % en poids;
d) le donneur de consistance en une concentration de 3-15 % en poids;
e) le porteur volatil cosmétiquement acceptable en une concentration de 30-60 % en poids;
f) l'agent auxiliaire de mise en suspension en une concentration de 1-7 % en poids; et
g) l'antioxydant en une concentration de 0,05-3 % en poids.

9. Un agent de stabilité à la lumière selon l'une quelconque des revendications 1 à 8, où sont présents:
a) le pigment ultrafin en une concentration de 10 % en poids;
b) le lipide en une concentration de 19 % en poids;
c) le co-lipide en une concentration de 10 % en poids;
d) le donneur de consistance en une concentration de 6 % en poids;
e) le porteur volatil cosmétiquement acceptable en une concentration de 53 % en poids;
f) l'agent auxiliaire de mise en suspension en une concentration de 2 % en poids; et
g) l'antioxydant en une concentration de 0,1 % en poids.

10. Un agent de stabilité à la lumière selon l'une quelconque des revendications 1 à 9, contenant 10 % en poids de dioxyde de titane ultrafin, 19 % en poids d'éthyl-hexyl-palmitate, 10 % en poids d'huile d'amande, 42,9 % en poids de cyclométicon, 10 % en poids de diméthicon, 3 % en poids de cire de carnauba, 3 % en poids de C₁₄-C₂₀ glycéride partiellement hydrogéné, 2 % en poids de dioxyde de silicium colloïdal et 0,1 % en poids de dl-alpha-tocophérol.
